# EUROPEAN PATENT SPECIFICATION

(11) **EP 2 308 819 B1**
(45) Date of publication and mention of the grant of the patent: **16.04.2014**
(21) Application number: 09771906.6
(22) Date of filing: 02.07.2009
(51) Int. Cl.: C07C 43/12, C07C 41/14, C07C 41/01, C07C 41/56, C07C 41/22

(54) **METHOD OF SYNTHESIZING SEVOFLURANE**
VERFAHREN ZUR SYNTHESE VON SEVOFLURAN
PROCÉDÉ DE SYNTHÈSE DE SÉVOFLURANE

(30) Priority: 02.07.2008 CN 200810129596
(43) Date of publication of application: 13.04.2011
(73) Proprietor: Lunan Pharmaceutical Group Corporation, Shandong Province 276006 (CN)
(72) Inventor: ZHAO, Zhiquan, Shandong 276005 (CN)
(74) Representative: Høiberg A/S
(86) International application number: PCT/CN2009/000750
(87) International publication number: WO 2010/000136

(56) References cited:
- WO-A2-2008/037040
- CN-A- 101 058 533
- CN-A- 101 381 289
- US-A- 5 811 596
- US-A- 6 100 434
- BIENIARZ, C. ET AL.: 'An efficient and environmentally friendly synthesis of the inhalation anesthetic sevoflurane' JOURNAL OF FLUORINE CHEMISTRY vol. 106, no. 1, 2000, pages 99 - 102, XP004218211

## Description

### Field of the Invention

The present invention relates to a method of synthesizing fluoromethyl-2,2,2-trifluoro-1-(trifluoromethyl)ethyl ether (known as sevoflurane).

### Background of the Invention

In recent years, it has been reported that fluorinated ethers exhibit potent inhaled anesthetic properties, Such kind of anesthetics includes desflurane (CF₃CHFOCHF₂), isoflurane (CF₃CHClOCHF₂), enflurane (ClFCHCF₂OCHF₂) and sevoflurane ((CF₃)₂CHOCH2F). Due to the short induction period and wake up period of sevoflurane, i.e. the desired characteristics for being an inhaled anesthetic, sevoflurane has been used as a very excellent inhaled anesthetic.

The US patents 3683092 and 3689571 have disclosed the use of sevoflurane as an inhaled anesthetic, and also a method of synthesizing sevoflurane, which involves reacting chloromethyl 2,2,2-trifluoro-1-(trifluoromethyl) ethyl ether with an excess amount of potassium fluoride in a high-boiling point solvent at 120°C to substitute chloromethyl with fluorine. These patents have also disclosed a synthesizing method, which involves reacting hexafluoro isopropanol and dimethyl sulfate with sodium hydroxide solution, followed by fluorinating the thus obtained methyl 2,2,2-trifluoro-1-(trifluoromethyl) ethyl ether using bromine trifluoride to produce sevoflurane. The US patent 4328376 has disclosed a method of separating sevoflurane from the olefinic side products produced by a method similar to that disclosed in US patent 3689571.

Other synthetic routes for sevoflurane can be found in the following publications: US patent 3897502, which involves fluorinating the methyl 2,2,2-trifluoro-1-(trifluoromethyl) ethyl ether using argon that contains 20% fluorine; US patent 4250334 and 4469898, which involves chloromethylating hexafluoro isopropanol using hydrogen fluoride, formaldehyde and sulfuric acid or other dehydrating agents; and PTC international application WO 97/25303, which involves the reaction between hexafluoro isopropanol and di(fluoromethyl) ether.

Okazaki et al. has disclosed a method that involves an electrochemical fluorination for yielding fluoromethyl ether (Fluorine Chemistry, 1974, 4(4), 387). The German Patent 25 20 962 has disclosed a method of synthesizing fluoromethyl ether from chloromethyl ether and hydrogen fluoride at 125°C -149°C in the presence of chromium oxyfluoride. Bensoam et al. [Tetrahedron Lett., 1979, 4, 353] has reported a method of synthesizing fluoromethyl ether by undergoing halogen exchange with tetrahydroxyfluoro phosphorane. The German Patent 2823 969 has disclosed a method of preparing organofluoride (including monofluoromethyl ether) from the reaction between the corresponding organochloride or bromide and a specifically selected ammonium hydrogenfluoride. Triethylamine hydrogenfluoride and pyridine hydrogenfluoride represent the typical examples of fluorinating agent for the preparation of said kind of organofluroide, typically with the yield of about 40-80% for fluorides. The Chinese patent 1244187 has made an improvement on this process with better result.

In addition, The US patent 4874901 has reported the reaction of chloromethyl 2,2,2-trifluoro-1-(trifluoromethyl) ethyl ether with pure potassium fluoride at high temperature and high pressure, of which, however, the reaction conversion is low.

The US patent 6100434 has reported a method of preparing sevoflurane using hexafluoro isopropanol as the staring material, in which chloromethyl 2,2,2-trifluoro-1-(trifluoromethyl) ethyl ether is firstly formed from hexafluoro isopropanol in the presence of anhydrous aluminum trichloride and trioxymethylene, and then chloromethyl 2,2,2-trifluoro-1-(trifluoromethyl) ethyl ether is reacted with a metal fluoride to substitute chlorine of chloromethyl with fluroine. Said method, however, has the following technical problems: (1) the first step of the reaction is a three-phase reaction, solidification occurs during the reaction, which makes stirring difficult, and readily leads to a bumping phenomenon in the post-treatment process which, in turn, causes a potential safety hazard; (2) the purity of the product from the first step {chloromethyl 2,2,2-trifluoro-1-(trifluoromethyl) ethyl ether} is low and the product contains lots of impurities.

PCT international publication WO2008037039 has disclosed a method of preparing sevoflurane from an intermediate, chloromethyl 2,2,2-trifluoro-1-(trifluoromethyl) ethyl ether produced by using hexafluoro isopropanol (C₃H₂F₆O, HFIP) as the starting material, and reacting HFIP directly with a formaldehyde equivalent, a strong acid and a chlorinating agent. Said method, however, has the following technical problems: (1) low conversion of hexafluoro isopropanol, and the treatment by pH adjusting for the recovery and purification of the unreacted hexafluoro isopropanol caused the production cost increased; (2) increased amount of impurities in the final products, which makes the purification process difficult (such as P3), and purity of sevoflurane become low due to the presence of impurities.

JOURNAL OF FLUORINE CHEMISTRY, vol. 106, no. 1, 2000, pages 99-102, discloses a method of synthesizing sevoflurane comprising reacting HFIP with (CH2O)n or trioxane in the presence of AlCl3 to give 2 and 3, followed by reaction with KF in PEG-400 to give sevoflurane.

US 5 811 596 A discloses a method of synthesizing sevoflurane comprising reacting paraformaldehyde and HFIPA in te presence of fuming sulfuric acid, followed by reaction with paraformaldehyde, HF and fuming sulfuric acid to give sevoflurane.

WO 2008/037040 A2 discloses a method of synthesizing sevoflurane comprising reacting HFIP and (CH2O)n in the presence of SOCI2, H2SO4 to give sevochlorane, followed by reaction with KF/KI to give sevoflurane.

### Summary of the Invention

In view of the drawbacks of the conventional method of synthesizing sevoflurane, the present invention provides a method of synthesizing sevoflurane which overcome all these drawbacks.

The objective of the present invention is to provide a method of synthesizing sevoflurane, and in comparison with the prior art, the synthesizing method of present invention improves the production yield of sevoflurane, lowers the production cost, and simplifies the production process.

The present invention provides a method of synthesizing sevoflurane, as defined in claim 1.

The method of synthesizing sevoflurane according to the present invention is illustrated in the following scheme:

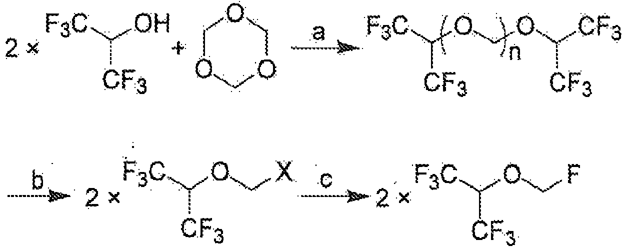

In the present invention, reaction of hexafluoro isopropanol with trioxymethylene or paraformaldehyde is conducted in an acidic environment; said acid is one or more acids selected from sulfuric acid, hydrochloric acid, phosphoric acid, chlorosulfonic acid, fluorosulfonic acid.

Hexafluoro isopropanol reacts with trioxymethylene or paraformaldehyde to form the dihexafluoro isopropanol formal derivatives, which are represented by the following structural formula (I):

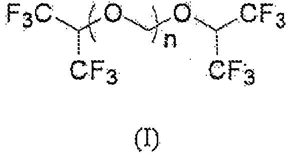

In formula (I), n is a natural number.

Typical examples of compound (I) include n=2 (i.e. dihexafluoro isopropanol diformal), n=3 (i.e. dihexafluoro isopropanol triformal), etc..

In the present invention, the main components of dihexafluoro isopropanol formal derivatives prepared from the reaction between hexafluoro isopropanol and trioxymethylene or paraformaldehyde are dihexafluoro isopropanol diformal and/or dihexafluoro isopropanol triformal.

In the present invention, reaction of dihexafluoro isopropanol formal derivatives and anhydrous aluminum trihalide can be conducted in the absence of a solvent or in the presence of a solvent. If the reaction is conducted in a solvent, the solvent is preferably selected from one or more of ethers, esters or halohydrocarbons, more preferably tetrahydrofuran, diethyl ether, ethyl acetate, chloroform or dichloromethane.

In the present invention, anhydrous aluminum trihalide used in the reaction between dihexafluoro isopropanol formal derivatives and anhydrous aluminum trihalide is anhydrous aluminum trichloride, anhydrous aluminum tribromide or anhydrous aluminum triiodide.

In the present invention, dihexafluoro isopropanol formal derivatives react with anhydrous aluminum trihalide to form halomethyl 2,2,2-trifluoro-1-(trifluoromethyl) ethyl ether of the following structural formula (II):

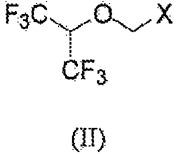

In formula (II), X is Cl, Br or I.

Typical examples of compound (II) includes X=Cl (i.e. chloromethyl 2,2,2-trifluoro-1-(trifluoromethyl) ethyl ether), X=Br (i.e. bromomethyl 2,2,2-trifluoro-1-(trifluoromethyl) ethyl ether) and X=I (i.e. iodomethyl 2,2,2-trifluoro-1-(trifluoromethyl) ethyl ether).

In the present invention, preferably, activated metal fluoride is reacted with halomethyl 2, 2, 2-trifluoro-1-(trifluoromethyl) ethyl ether in the presence of an activating agent. Preferably, said activating agent is ethylene glycol, diethylene glycol, triethylene glycol or 18-crown-6, and more preferably, said activating agent is triethylene glycol.

In the present invention, reaction of halomethyl 2,2,2-trifluoro-1-(trifluoromethyl) ethyl ether with metal fluoride is conducted in the absence of a solvent or in the presence of a solvent. If the reaction is conducted in the present of a solvent, the solvent is preferably an inert high-boiling point solvent, more preferably, the solvent is acetamide, sulfolane or N,N-dimethylformamide.

In the present invention, metal fluoride used in the reaction between halomethyl 2,2,2-trifluoro-1-(trifluoromethyl) ethyl ether and metal fluoride is preferably potassium fluoride, sodium fluoride. Alternatively ammonium fluoride can be used.

In one embodiment of the present invention, a method of synthesizing sevoflurane comprises the following steps:
a) hexafluoro isopropanol and trioxymethylene or paraformaldehyde solution are subjected to condensation reaction in the presence of an acid at a temperature of 0°C∼100°C for 4∼48 hours. After the reaction, the reaction system is left aside for phase separation to remove the acid, and then cooled to -5°C∼5°C, crystals formed while stirring and kept the crystal formation for 1∼8 hours, and then filtered to obtain the solid retentate. The retentate is left to dry for later use. The filtrate is a solution of hexafluoro isopropanol, trioxymethylene or paraformaldehyde from incomplete conversion. After filtration, said filtrate can be used directly and repeatedly.
b) Retentate from step a) is mixed with anhydrous aluminum trihalide, and reaction is conducted at 0°C∼60°C for 4-48 hours to give halomethyl 2,2,2-trifluoro-1-(trifluoromethyl)ethyl ether.
c) Activated metal fluoride is reacted with halomethyl 2,2,2-trifluoro-1-(trifluoromethyl) ethyl ether from step b) in the presence of an activating agent to give the crude sevoflurane (in accordance with the Chinese patent ZL200510071849.9), and purified sevoflurane is obtained after distillation.

In another embodiment of the present invention, a method of synthesizing sevoflurane comprises the following steps:
a) Hexafluoro isopropanol and trioxymethylene or paraformaldehyde solution are subjected to condensation reaction in the presence of an acid at 35°C∼45°C for 4∼6 hours. After the reaction, the reaction system is left aside for phase separation to remove the acid, and then cooled to -5°C∼0°C, crystal formed while stirring and kept the crystal formation for 4∼8 hours, and filtered to obtain the solid retentate. The retentate is left to dry for later use. The filtrate is a solution of hexafluoro isopropanol, trioxymethylene or paraformaldehyde from incomplete conversion. After filtration, said filtrate can be used directly and repeatedly.
b) Retentate from step a) is mixed with anhydrous aluminum trihalide, and reaction is conducted at 25°C∼35°C for 10∼15 hours to give halomethyl 2,2,2-trifluoro-1-(trifluoromethyl) ethyl ether.
c) Activated metal fluoride is reacted with halomethyl 2,2,2-trifluoro-1-(trifluoromethyl) ethyl ether from step b) in the presence of an activating agent to give the crude sevoflurane (in accordance with the Chinese patent ZL200510071849.9), and purified sevoflurane is obtained after distillation.

The method of preparing sevoflurane provided in the present invention involves the preparation of monohalomethyl ether from hexafluoro isopropanol via dihexafluoro isopropanol formal derivatives, from which sevoflurane is prepared. Said method is a noval method that has never been reported. Said method possesses the following advantages: (1) After the reaction, unreacted hexafluoro isopropanol and paraformaldehyde solution can be used directly and repeatedly after filtration, and no need of purification process for recovering; (2) intermediate, i.e. dihexafluoro isopropanol formal derivatives are in solid form, which facilitates the process of purification, storage and transportation, and impurities with low-boiling point can be removed easily; (3) stirring in the entire process is improved, operation become simplified, and is of lower risk; and the intermediate, i.e. halomethyl 2,2,2-trifluoro-1-(trifluoromethyl) ethyl ether is obtained with higher purity and higher production yield; (4) the method of synthesizing sevoflurane of the present invention possesses the advantages of lower consumption of starting materials, simple and controllable operation process, less three-wastes (waste solid, waste water and waste gas), lower production cost, higher conversion, readily achievable reaction condition, which is suitable for large scale industrial production.

### Preferred embodiments of the Invention

By making reference to the following examples, the present invention will become more apprehended to the ordinary person in the art. However, the examples are only for illustrative purpose, but not intended to constrain the present invention in any ways.

### Step 1) : preparation of the intermediate:

### Example 1

Hexafluoro isopropanol (336g), trioxymethylene (90g), and concentrated sulfuric acid (15ml) were placed in a reaction flask, stirred and heated to 40°C∼45° C for 6 hours, left aside for phase separation, followed by removal of the concentrated sulfuric acid, stirred and cooled to 0° C or so, crystals formed and kept for 4 hours, and then filtered to give 149.6g filtrate, which was then subjected to gas chromatography (GC) analysis and a mixture of hexafluoro isopropanol and trioxymethylene was confirmed. After simple filtration, said mixture was directly and repeatedly used for reaction; while the retentate obtained from the filtration was dried to give a solid of 255.5g. Said retentate was subjected to GC analysis and confirmed as dihexafluoro isopropanol triformal (n=3) content: 41%, dihexafluoro isopropanol diformal (n=2) content: 53%, dihexafluoro isopropanol monoformal (n=1) content: <0.5%.

### Example 2

Hexafluoro isopropanol (336g), trioxymethylene (90g), concentrated sulfuric acid (15ml) were placed in a reaction flask, stirred and heated to 35°C∼40°C for 4 hours, left aside for phase separation, followed by removal of the concentrated sulfuric acid, stirred and cooled to 0° C or so, crystals formed and kept for 4 hours, and then filtered to give 178.8g filtrate, which was then subjected to gas chromatography (GC) analysis and a mixture of hexafluoro isopropanol and trioxymethylene was confirmed. After simple filtration, said mixture was directly and repeatedly used for reaction, while the retentate obtained from the filtration was dried to give a solid of 250.1g. Said retentate was subjected to GC analysis and confirmed as dihexafluoro isopropanol triformal (n=3) content: 42%, dihexafluoro isopropanol diformal (n=2) content: 52%, dihexafluoro isopropanol monoformal (n=1) content:<0.5%.

### Example 3

Hexafluoro isopropanol (336g), paraformaldehyde (90g), concentrated hydrochloric acid (45ml) were placed in a reaction flask, stirred and heated to 0°C∼ 5°C for 48 hours, left aside for phase separation, followed by removal of the concentrated hydrochloric acid, stirred and cooled to -5°C or so, crystals formed and kept for 8 hours, and then filtered to give 162.9g filtrate, which was then subjected to gas chromatography (GC) analysis and a mixture of hexafluoro isopropanol and trioxymethylene was confirmed. After simple filtration, said mixture was directly and repeatedly used for reaction, while the retentate obtained from the filtration was dried to give a solid of 235.6g. Said retentate was subjected to GC analysis and confirmed as dihexafluoro isopropanol triformal (n=3) content: 49%, dihexafluoro isopropanol diformal (n=2) content: 44%, dihexafluoro isopropanol monoformal (n=1) content:<0.2%.

### Example 4

Hexafluoro isopropanol (336g), trioxymethylene (90g), concentrated phosphoric acid (15ml) were placed in a reaction flask, stirred and heated to 95°C∼100°C for 4 hours, left aside for phase separation, followed by removal of the concentrated phosphoric acid, stirred and cooled to 5°C or so, crystals formed and kept for 1 hour, and then filtered to give 172.7g filtrate, which was then subjected to gas chromatography (GC) analysis and a mixture of hexafluoro isopropanol and trioxymethylene was confirmed. After simple filtration, said mixture was directly and repeatedly used for reaction, while the retentate obtained from the filtration was dried to give a solid of 230.7g. Said retentate was subjected to GC analysis and confirmed as dihexafluoro isopropanol triformal (n=3) content: 38%, dihexafluoro isopropanol diformal (n=3) content: 55%, dihexafluoro isopropanol monoformal (n=1) content: <0.9%.

### Step 2) : Chloromethylation of the intermediate:

### Example 5

123.6g solid obtained from Example I was weighed and placed in a reaction flask, which was then heated to 40°C∼45°C, upon the solid was melted, reaction temperature was lowered to 20°C∼25°C, followed by the addition of anhydrous aluminum trichloride (84.5g) in portions, temperature was kept at 25°C∼30°C for 10 hours, and then cooled to 0°C or so, which was then treated with dropwise addition of 10% diluted hydrochloric acid (500ml), and left aside for phase separation, the organic layer was sequentially washed with 5% NaOH aqueous solution (100ml×2), water (100ml×2), dried over anhydrous sodium sulfate, to give 111.6g chloromethyl 2,2,2-trifluoro-1-(trifluoromethyl) ethyl ether, purity: 98.7% (GC analysis).

### Example 6

123.6g solid obtained from Example 1 was weighed and placed in a reaction flask, followed by the addition of 100ml dichloromethane, and anhydrous aluminum trichloride (84.5g) was added at 0°C in portions, reaction temperature was kept at 30°C∼35°C for 15 hours, and then cooled to 0°C, which was then treated with dropwise addition of 10% diluted hydrochloric acid (530ml), and left aside for phase separation, the organic layer was sequentially washed with 5% NaOH aqueous solution (100ml×2), water (100ml×2), dried over anhydrous sodium sulfate, and filtered. Filtrate was subjected to distillation to remove dichloromethane to give 113.3g of chloromethyl 2,2,2-trifluoro-1-(trifluoromethyl) ethyl ether, purity: 98.1 %(GC analysis).

### Example 7

123.6 g solid obtained from Example 1 was weighed and placed in a reaction flask, followed by the addition of 100ml chloroform, upon the solid was dissolved, anhydrous aluminum tribromide (168.5 g) was added at 0-5°C in portions, reaction temperature was kept at 55°C∼60°C for 4 hours, and then cooled to 0 °C, which was then treated with dropwise addition of 10% diluted hydrobromic acid (550 ml), and left aside for phase separation, the organic layer was sequentially washed with 5% NaOH aqueous solution (100ml×2), water (100ml×2), dried over anhydrous sodium sulfate, chloroform was removed by distillation to give 126.6g of bromomethyl 2,2,2-trifluoro-1-(trifluoromethyl) ethyl ether, purity: 98.8%(GC analysis).

### Example 8

123.6 g solid obtained from Example 1 was weighed and placed in a reaction flask, followed by the addition of 100 ml dichloromethane, upon the solid was dissolved, anhydrous aluminum triiodide (257.5 g) was added at 0-5 °C in portions, reaction temperature was kept at 0°C∼5°C for 48 hours, and then cooled to 0 °C, which was then treated with dropwise addition of 10% diluted hydroiodic acid (600 ml), and left aside for phase separation, the organic layer was sequentially washed with 5% NaOH aqueous solution (100ml×2), water (100ml×2), dried over anhydrous sodium sulfate, dichloromethane was removed by distillation to give 146,7 g of iodomethyl 2, 2, 2-trifluoro-1-(trifluoromethyl) ethyl ether, purity: 98.2% (GC analysis).

### Step 3): Preparation of sevoflurane:

### Example 9

Chloromethyl 2,2,2-trifluoro-1-(trifluoromethyl) ethyl ether (500.0g), highly active anhydrous potassium fluoride (140.0g), acetamide (500.0g) and triethylene glycol (60.0g) were placed in a 1000mL single-neck flask provided with a magnetic stirring bar and reflux apparatus, and the reaction mixture was heated to 85°C and reflux for 6 hours. After the reaction, crude sevoflurane (388.1g) was obtained from distillation, yield: 84.0%, purity: 97.8% (GC analysis).

Crude sevoflurane (388.1g) was placed and subjected to distillation in a distillation apparatus to give sevoflurane of qualified grade (345.8g), purity: 99.998% (GC analysis).

### Example 10

Bromomethyl 2,2,2-trifluoro-1-(trifluoromethyl) ethyl ether (520.0g), highly active anhydrous potassium fluoride (130.0g), sulfolane (500.0g) and triethylene glycol (60.0g) were placed in a 1000mL single-neck flask provided with a magnetic stirring bar and reflux apparatus, and the reaction mixture was heated to 85°C and reflux for 6 hours. After the reaction, crude sevoflurane (334.4g) was obtained from distillation, yield: 83.6%, purity: 98.7% (GC analysis).

Crude sevoflurane (334.4g) was placed and subjected to distillation in a distillation apparatus to give sevoflurane of qualified grade (292.9g), purity: 99.997% (GC analysis).

### Example 11

Iodomethyl 2,2,2-trifluoro-1-(trifluoromethyl) ethyl ether (620.0g), highly active anhydrous potassium fluoride (125.0g), N,N-dimethylformamide (500.0g) and triethylene glycol (60.0g) were placed in a 1000mL single-neck flask provided with a magnetic stirring bar and reflux apparatus, and the reaction mixture was heated to 85°C and reflux for 6 hours. After the reaction, crude sevoflurane (331.7g) was obtained from distillation, yield: 82.4%, purity: 98.3% (GC analysis).

Crude sevoflurane (331.7g) was placed and subjected to distillation in a distillation apparatus to give sevoflurane of qualified grade (286.1g), purity: 99.997% (GC analysis).

The method of synthesizing sevoflurane of the present invention has the advantages of high product quality (high purity), high production yield, ease of removal of impurities, simple purification process for recovering starting materials, low starting material consumption, which is suitable for large scale industrial production.

The present invention has been described in details in accordance with the specified embodiments as set forth.

## Claims

1. A method of synthesizing sevoflurane comprising the following steps:
1) Subjecting hexafluoro isopropanol and trioxymethylene or paraformaldehyde to condensation reaction in the presence of an acid to give a solid form of dihexafluoro isopropanol formal derivatives, wherein said acid is one or more acids selected from sulfuric acid, hydrochloric acid, phosphoric acid, chlorosulfonic acid and fluorosulfonic acid;
2) To the thus obtained dihexafluoro isopropanol formal derivatives, adding an anhydrous aluminum trihalide to prepare the halomethyl 2, 2, 2-trifluoro-1-(trifluoromethyl) ethyl ether, wherein the anhydrous aluminum trihalide is anhydrous aluminum trichloride, anhydrous aluminum tribromide or anhydrous aluminum triiodide; and
3) Reacting the halomethyl 2,2,2-trifluoro-1-(trifluoromethyl) ethyl ether obtained from 2) with a metal fluoride or ammonium fluoride to provide sevoflurane.

2. The method according to claim 1, wherein said metal fluoride in step 3) is metal fluoride activated in the presence of an activating agent selected from the group consisting of ethylene glycol, diethylene glycol, triethylene glycol or 18-crown-6.

3. The method according to claim 2, wherein step 1) is conducted at a temperature range from 0°C∼100°C for 4-48 hours; step 2) is conducted at a temperature range from 0°C ∼60°C for 4-48 hours.

4. The method according to claim 3, wherein after step 1), the temperature is cooled to -5°C∼5°C, stirred, crystals formed and kept the crystal formation for 1∼8 hours.

5. The method according to claim 1, wherein said dihexafluoro isopropanol formal derivatives obtained in step 1) are represented by the following structural formula: 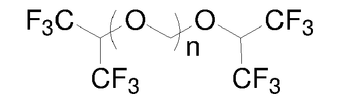 wherein, n is an integer equal to or greater than 1.

6. The method according to claim 5, wherein the main components of said dihexafluoro isopropanol formal derivatives obtained in step 1) are dihexafluoro isopropanol diformal and/or dihexafluoro isopropanol triformal.

7. The method according to claim 1, wherein in step 2), reaction of dihexafluoro isopropanol formal derivatives and anhydrous aluminum trihalide is conducted in a solvent selected from one or more of an ether, an ester and a halohydrocarbon.

8. The method according to claim 1, wherein said halomethyl 2,2,2-trifluoro-1-(trifluoromethyl) ethyl ether obtained in step 2) is represented by the following structural formula: 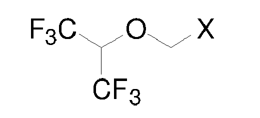 wherein, X is any one of Cl, Br or I.

9. The method according to claim 1, wherein step 3) is performed in an inert high-boiling point solvent.

10. The method according to claim 9, wherein said inert high-boiling point solvent is selected from acetamide, sulfolane or N, N-dimethylformamide.

11. The method according to claim 1, wherein said metal fluoride in step 3) is selected from potassium fluoride or sodium fluoride.

## Patentansprüche

1. Verfahren zur Synthese von Sevofluran, aufweisend die folgenden Schritte:
1) Zuführen von Hexafluorisopropanol und Trioxymethylen oder Paraformaldehyd einer Kondensationsreaktion in Gegenwart einer Säure, um eine feste Form von Dihexafluorisopropanol-Formalderivaten zu erhalten, wobei es sich bei der Säure um eine oder mehrere Säuren handelt, die aus einer Gruppe bestehend aus Schwefelsäure, Chlorwasserstoffsäure, Phosphorsäure, Chlorsulfonsäure und Fluorsulfonsäure ausgewählt sind,
2) Hinzufügen zu den somit erhaltenen Dihexafluorisopropanol-Formalderivaten eines anhydrischen Aluminium-Trihalogenids, um den Halogenmethyl 2, 2, 2-trifluor-1-(Trifluormethyl)ethylether bereitzustellen, wobei das anhydrische Aluminium-Trihalogenid ein anhydrisches Aluminium-Trichlorid, ein anhydrisches AluminiumTribromid, oder ein anhydrisches Aluminium-Triiodit ist, und
3) Reagieren des Halogenmethyl 2,2,2-trifluor-1-(Trifluormethyl)ethylethers, der in Schritt 2) erhalten wurde, mit einem Metallfluorid oder einem Ammoniumfluorid, um Sevofluran zu erhalten.

2. Verfahren nach Anspruch 1, bei dem das Metallfluorid in Schritt 3) ein Metallfluorid ist, das in Gegenwart eines Aktivierungsmittels, das aus einer Gruppe bestehend aus Ethylenglykol, Diethylenglykol, Triethylenglykol oder 18-Crown-6 ausgewählt wird, aktiviert wird.

3. Verfahren nach Anspruch 2, bei dem Schritt 1) in einem Temperaturbereich von 0 °C ∼ 100 °C für 4 - 48 Stunden, und bei dem Schritt 2) in einem Temperaturbereich von 0 °C ∼ 60 °C für 4 ∼ 48 Stunden durchgeführt wird.

4. Verfahren nach Anspruch 3, bei dem nach Schritt 1) die Temperatur auf -5 °C ∼ 5 °C herabgekühlt wird, gerührt wird, Kristalle gebildet werden und die Kristallformation für 1 - 8 Stunden beibehalten wird.

5. Verfahren nach Anspruch 1, bei dem die in Schritt 1) erhaltenen Dihexafluorisopropanol-Formalderivate durch die folgende Strukturformel dargestellt werden: 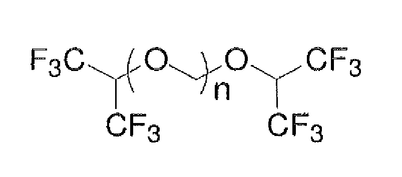 wobei n eine ganze Zahl gleich oder größer als 1 ist.

6. Verfahren nach Anspruch 5, bei dem die Hauptbestandteile der in Schritt 1) erhaltenen Dihexafluorisopropanol-Formalderivate Dihexafluorisopropanol-Diformal und/oder Dihexafluorisopropanol-Triformal sind.

7. Verfahren nach Anspruch 1, bei dem in Schritt 2) eine Reaktion von Dihexafluorisopropanol-Formalderivaten und anhydrischen Aluminium-Trihalogeniden in einer Lösung durchgeführt wird, die ausgewählt wird aus einer oder mehreren einer Gruppe bestehend aus Ether, Ester und Halogenkohlenwasserstoff.

8. Verfahren nach Anspruch 1, bei dem der in Schritt 2) erhaltene Halogenmethyl 2, 2, 2-trifluor-1-(Trifluormethyl)ethylether durch die folgende Strukturformel dargestellt wird: 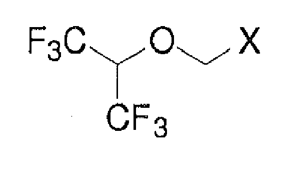 wobei X eines von Cl, Br oder I ist.

9. Verfahren nach Anspruch 1, bei dem Schritt 3) in einem reaktionsträgen hochsiedenden Lösungsmittel durchgeführt wird.

10. Verfahren nach Anspruch 9, bei dem das reaktionsträge hochsiedende Lösungsmittel ausgewählt wird aus einer Gruppe bestehend aus Acetamid, Sulfolan oder N, N-Diemethylformamid.

11. Verfahren nach Anspruch 1, bei dem das Metallfluorid in Schritt 3) aus einer Gruppe bestehend aus Kaliumfluorid oder Natriumfluorid ausgewählt wird.

## Revendications

1. Procédé de synthèse de sévoflurane comprenant les étapes suivantes :
1) le fait de soumettre de l'hexafluoro isopropanol et du trioxyméthylène ou paraformaldéhyde à une réaction de condensation en présence d'un acide afin de donner une forme solide de dérivés dihexafluoro isopropanol formal, dans lequel ledit acide est un ou plusieurs acides choisis parmi l'acide sulfurique, l'acide chlorhydrique, l'acide phosphorique, l'acide chlorosulfonique et l'acide fluorosulfonique ;
2) aux dérivés dihexafluoro isopropanol formal ainsi obtenus, l'ajout d'un trihalogénure d'aluminium anhydre afin de préparer l'halogénométhyl 2,2,2-trifluoro-1-(trifluorométhyl)éthyléther, dans lequel le trihalogénure d'aluminium anhydre est le trichlorure d'aluminium anhydre, le tribromure d'aluminium anhydre ou le triiodure d'aluminium anhydre ; et
3) la mise en réaction de l'halogénométhyl 2,2,2-trifluoro-1-(trifluorométhyl)éthyléther obtenu en 2) avec un fluorure de métal ou du fluorure d'ammonium afin de former le sévoflurane.

2. Procédé selon la revendication 1, dans lequel ledit fluorure de métal à l'étape 3) est un fluorure de métal activé en présence d'un agent activant choisi dans le groupe consistant en l'éthylène glycol, le diéthylène glycol, le triéthylène glycol ou le 18-couronne-6.

3. Procédé selon la revendication 2, dans lequel l'étape 1) est conduite à une plage de températures de 0 °C à 100 °C pendant 4 à 48 heures ; l'étape 2) est conduite à une plage de températures de 0 °C à 60 °C pendant 4 à 48 heures.

4. Procédé selon la revendication 3, dans lequel, après l'étape 1), la température est refroidie à -5 °C à 5 °C, on réalise une agitation, des cristaux se forment et on maintient la formation de cristaux pendant 1 à 8 heures.

5. Procédé selon la revendication 1, dans lequel lesdits dérivés dihexafluoro isopropanol formal obtenus à l'étape 1) sont représentés par la formule développée suivante : 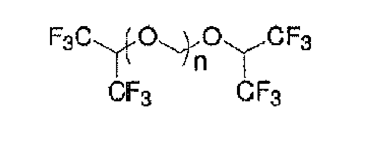 dans laquelle, n est un nombre entier supérieur ou égal à 1.

6. Procédé selon la revendication 5, dans lequel les composants principaux desdits dérivés dihexafluoro isopropanol formal obtenus à l'étape 1) sont le dihexafluoro isopropanol diformal et/ou le dihexafluoro isopropanol triformal.

7. Procédé selon la revendication 1, dans lequel à l'étape 2), la réaction de dérivés dihexafluoro isopropanol formal et de trihalogénure d'aluminium anhydre est conduite dans un solvant choisi parmi un ou plusieurs d'un éther, un ester et un halogénohydrocarbure.

8. Procédé selon la revendication 1, dans lequel ledit halogénométhyl 2,2,2-trifluoro-1-(trifluorométhyl)éthyléther obtenu à l'étape 2) est représenté par la formule développée suivante : 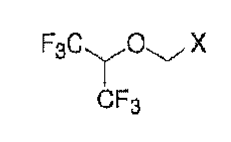 dans laquelle, X est l'un quelconque de Cl, Br ou I.

9. Procédé selon la revendication 1, dans lequel l'étape 3) est réalisée dans un solvant inerte à point d'ébullition élevé.

10. Procédé selon la revendication 9, dans lequel ledit solvant inerte à point d'ébullition élevé est choisi parmi l'acétamide, le sulfolane ou le N,N-diméthylformamide.

11. Procédé selon la revendication 1, dans lequel ledit fluorure de métal à l'étape 3) est choisi parmi le fluorure de potassium ou le fluorure de sodium.
